# EUROPEAN PATENT SPECIFICATION

(11) **EP 0 513 861 B1**
(45) Date of publication and mention of the grant of the patent: **26.02.1997**
(21) Application number: 92113037.3
(22) Date of filing: 02.11.1988
(51) Int. Cl.: A61K 39/39

(54) **Vaccine adjuvant comprising a tetra-polyol**
Einen Tetra-Polyol enthaltendes Impfstoff-Adjuvans
Adjuvant pour vaccin comprenant un tetra-polyol

(30) Priority: 03.11.1987 US 116425
(43) Date of publication of application: 19.11.1992
(62) Divisional of application: 88118263.8
(73) Proprietor: SYNTEX (U.S.A.) INC., Palo Alto California 94304 (US)
(72) Inventor: Allison, Anthony Clifford, Belmont, CA 94002 (US); Byars, Noelene Elva, Sunnyvale, CA 94087 (US); Fu, Cherng-Chyi, Saratoga, CA 95070 (US); Lidgate, Deborah Marilyn, Los Altos, CA 94022 (US); Felgner, Philip Lewis, Rancho Sante Fe, CA 92067 (US); Foster, Linda Cheryl, Sunnyvale, CA 94086 (US); Lee, William Alfred, Los Altos, CA 94022 (US)
(74) Representative: Witte, Hubert

(56) References cited:
- EP-A- 0 135 376
- WO-A-88/01873
- JOURNAL OF IMMUNOLOGY, vol. 127, no. 3, 03 September 1981, Baltimore, MD (US); R. HUNTER et al., pp. 1244-1250
- VACCINE, vol. 5, no. 3, September 1987, Guildford (GB); N.E. BYARS et al., pp. 223-228

## Description

This invention relates to improved vaccine adjuvant formulations, improved processes for preparing said adjuvant formulations, and methods of using the improved formulations.

Adjuvants are useful for improving the immune response obtained with any particular antigen in a vaccine. Although some antigens are administered in vaccines without an adjuvant, there are many antigens that lack sufficient immunogenicity to stimulate a useful immune response in the absence of an effective adjuvant. Adjuvants also improve the immune response obtained from "self-sufficient" antigens, in that the immune response obtained may be increased or the amount of antigen administered may be reduced.

The standard adjuvant for use in laboratory animals is Freund's adjuvant. Freund's complete adjuvant (FCA) is an emulsion containing mineral oil and killed mycobacteria in saline. Freund's incomplete adjuvant (FIA) omits the mycobacteria. Both FIA and FCA induce exceptional humoral (antibody) immunity, and FCA additionally induces high levels of cell-mediated immunity. However, neither FIA nor FCA are acceptable for use outside the laboratory due to the adjuvants' side effects. Mineral oil is known to cause abscesses and granulomas, while Mycobacterium tuberculosis is the agent responsible for tuberculosis.

A number of naturally occurring compounds such as the lipid-A portion of gram negative bacteria endotoxin and trehalose dimycolate of mycobacteria have been tried as substitutes for FCA and FIA. Also, the phospholipid lysolecithin has been shown to have adjuvant activity (B. Arnold et al., Eur. J. Immunol., 9:363-366 (1979)). In addition, several synthetic surfactants, for example, dimethyldioctadecyl ammonium bromide (DDA) and certain linear polyoxypropylene-polyoxyethylene (POP-POE) block polymers (available commercially under the trademark Pluronic®) have been reported as having adjuvant activity (H. Snippe et al, Int. Archs. Allergy Appl. Immun., 65, 390-398 (1981)). R. Hunter et al. have reported in J. Immunol., 127, 1244-1250 (1981) that POP-POE block polymers increase antibody formation to bovine serum albumin (BSA) in mice when used as the surfactant component of an mineral oil/water emulsion adjuvant formulation. While these natural and synthetic surfactants demonstrate some degree of adjuvanticity, they for the most part fail to achieve the degree of immunopotentiation obtained using FCA or FIA.

Taking another approach, it has been determined that the adjuvant effect from mycobacteria is due to a muramyl-peptide in the cell wall. The smallest fragment of this molecule that retains adjuvant activity is N-acetylmuramyl-L-alanyl-D-isoglutamine, commonly known as muramyldipeptide or "MDP" (Ellouz et al, Biochem. & Biophys. Res. Comm., Vol 59, 4, 1317 (1974)). Numerous analogs of MDP have been prepared, and are also referred to as "MDPs." See for example Audibert et al., U.S. Pat. No. 4,158,052; Audibert et al., U.S Pat. No. 4,220,637; Audibert et al., U.S. Pat. No. 4,323,559; Baschang et al., U.S. Pat. No. 4,323,560; Baschang et al., U.S. Pat. No. 4,409,209; Baschang et al., U.S. Pat. No. 4,423,038; Derrien et al., U.S. Pat. No. 4,185,089; Hartmann et al., U.S Pat. No. 4,406,889; Jones et al., U.S. Pat. No. 4,082,735; Jones et al., U.S. Pat. No. 4,082,736; Le Francier et al., U.S. Pat. No. 4,427,659; Le Francier et al., U.S. Pat. No. 4,461,761; Yamamura et al., U.S. Pat. No. 4,314,998; Yamamura et al., U.S. Pat. No. 4,101,536; and Yamamura et al., U.S. Pat. No. 4,369,178. While these compounds are weakly effective at stimulating the immune system when administered in aqueous solution, the results generally fall short of the specific immune response obtained with FIA or FCA.

A particularly effective adjuvant formulation comprising a glycopeptide, a non-toxic POP-POE block polymer, a glycol ether-based surfactant, a metabolizable oil, and buffered saline was recently described by Allison et al., U.S. Pat. No. 4,606,918. This formulation is capable of inducing strong humoral and cell-mediated immune responses, equivalent or superior to the results achieved in laboratory animals using FCA. However, the formulation is prone to instability and separation (e.g., creaming) upon standing. We have discovered that upon refrigeration it loses its ability to potentiate the primary response to antigens. Also, it has been found difficult to prepare a stable, homogenous emulsion with retention of full adjuvant activity on a commercial scale.

We have now discovered that an immunopotentiating glycopeptide can be formulated with a non-toxic N,N,N',N'-tetra(polyoxypropylene-polyoxyethylene)-1,2-diaminoethane block polymer ("tetra-polyol"), resulting in an adjuvant emulsion that overcomes certain problems of the prior art, for example, toxicity and failure to stimulate cell-mediated immunity. This new adjuvant has activity equal or greater than the activity of FCA and is significantly more effective for increasing the immunogecity of antigens than Allison's composition. The formulation of the present invention is easily manufactured with full retention of activity, and displays greater stability than Allison's composition. Because the tetra-polyol is non-toxic, this adjuvant formulation may be safely used as a vehicle for enhancing the immunogenicity of antigens administered to birds and mammals.

We have also invented a particularly advantageous method for preparing adjuvant emulsions, using tetra-polyols, which maintains the formulations' efficacy, enhances its physical stability, and reduces its sensitivity to refrigeration. Remarkably, such adjuvant emulsions may even be frozen and still retain efficacy.

One aspect of the invention is an adjuvant in the form of an emulsion having oily particles dispersed in a continuous aqueous phase, for potentiating the immunogenicity of an antigen, which adjuvant comprises:
an emulsion-forming amount of a non-toxic tetra-polyol; and
an immunopotentiating amount of a glycopeptide.

Another aspect of the invention is an adjuvant in the form of an emulsion having oily particles dispersed in a continuous aqueous phase, for potentiating the immunogenicity of an antigen, which adjuvant comprises an emulsion-forming amount of a non-toxic tetra-polyol; optionally, an emulsion-forming amount of a non-toxic metabolizable oil; optionally, an emulsion-stabilizing amount of a glycol ether-based surfactant; water or aqueous solution, and an immunopotentiating amount of a muramyldipeptide, preferably a derivative of formula I 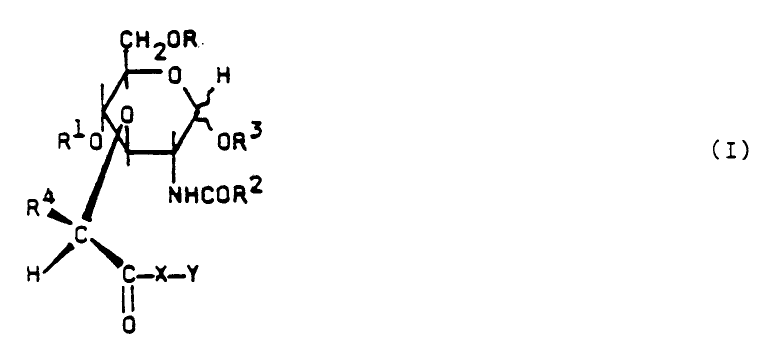 and the pharmaceutically acceptable salts thereof, wherein R and R₁ are each independently H or acyl of 1 to 22 carbon atoms, R₂ is alkyl or aryl, optionally substituted with halo, nitro, or lower alkyl, R₃ is H, alkyl, or aryl, R₄ is H or lower alkyl, X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl, and Y is D-glutamine, D-isoglutamine or D-isoasparagine.

Another aspect of the invention is a vaccine, comprising an adjuvant of the invention in combination with an immunogenic amount of an antigen.

Another aspect of the invention is a process for preparing an adjuvant of the invention, which process comprises preparing a first mixture comprising the polymer, oil, surfactant, and water or aqueous solution; emulsifying the mixture to produce an oil-in-water type emulsion having oily particles dispersed in a continuous aqueous phase; and combining the emulsion with a muramyldipeptide derivative of formula I.

Another aspect of the invention is a kit for extemporaneous preparation of an adjuvant of the invention, which kit comprises: a first container containing an emulsion as described above, and a second container containing the muramyl dipeptide, preferably N-acetylmuramyl-L-threonyl-D-isoglutamine optionally in an aqueous solution or suspension, where the concentrations of the components in each container are selected such that combination of the contents of both containers produces an adjuvant of the invention.

Another aspect of the invention is a kit for the preparation of a vaccine of the invention, which differs from the adjuvant kit described above in that an immunogenic amount of an antigen is added to the second container, or present in a third container.

One aspect of the invention is an adjuvant in the form of an emulsion having oily particles dispersed in a continuous aqueous phase, for potentiating the immunogenicity of an antigen, which adjuvant comprises:
an emulsion-forming amount of a non-toxic tetra-polyol; and
an immunopotentiating amount of a glycopeptide.

Another aspect of the invention is an adjuvant in the form of an emulsion having oily particles dispersed in a continuous aqueous phase, for potentiating the immunogenicity of an antigen, which adjuvant comprises an emulsion-forming amount of a non-toxic tetra-polyol; optionally, an emulsion-forming amount of a non-toxic metabolizable oil; optionally an emulsion-stabilizing amount of a glycol ether-based surfactant; water or aqueous solution, and an immunopotentiating amount of a muramyldipeptide, preferably a derivative of formula I 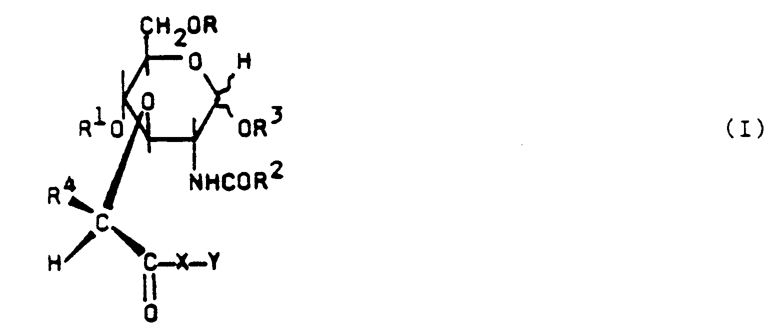 and the pharmaceutically acceptable salts thereof, wherein R and R₁ are each independently H or acyl of 1 to 22 carbon atoms, R₂ is alkyl or aryl, optionally substituted with halo, nitro, or lower alkyl, R₃ is H, alkyl, or aryl, R₄ is H or lower alkyl, X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl, and Y is D-glutamine, D-isoglutamine or D-isoasparagine. A preferred subgenus is the adjuvant wherein said tetra-polyol is Tetronic® 1501, particularly where said muramyldipeptide derivative of formula I is N-acetylmuramyl-L-threonyl-D-isoglutamine. A preferred class of the invention is the adjuvant which includes a-non-toxic metabolizable oil, especially where said oil is squalene or squalane. A preferred subclass is the adjuvant wherein said glycol ether-based surfactant is Tween® 80, particularly where said water or aqueous solution comprises isotonic buffered saline.

Another preferred subgenus is the adjuvant wherein said tetra-polyol is Tetronic® 1501 and said muramyldipeptide derivative of formula I is murabutide.

Another aspect of the invention is an adjuvant in the form of an oil-in-water type emulsion, having oily particles dispersed in a continuous aqueous phase, for potentiating the immunogenicity of an antigen, which adjuvant comprises a non-toxic tetra-polyol in an amount of 0.2 to 49%; a non-toxic metabolizable oil in an amount of 0-15%; a glycol ether-based surfactant in an amount of 0.05-5%; water or aqueous solution; and 0.0001-10% of a muramyldipeptide derivative of formula I (% are vol./vol., except for the muramyldipeptide which is wt./vol.). A preferred subgenus is the adjuvant wherein said tetra-polyol is Tetronic® 1501, particularly where said muramyldipeptide derivative of formula I is N-acetyl-muramyl-L-threonyl-D-isoglutamine. A preferred class is the adjuvant which includes a non-toxic metabolizable oil, wherein said oil is squalene or squalane. A presently preferred embodiment of the invention is the adjuvant in the form of an oil-in-water type emulsion, having oily particles dispersed in a continuous aqueous phase, for potentiating the immunogenicity of an antigen, which adjuvant comprises Tetronic® 1501 in an amount of 1-10%; squalane or squalene in an amount of 1-10%; Tween® 80 in an amount of about 0.2%; isotonic buffered saline (using phosphate buffers, acetate buffers, or combinations thereof); and 0.0001-10% N-acetylmuramyl-L-threonyl-D-isoglutamine.

Another aspect of the invention is a vaccine comprising an adjuvant of the invention in combination with an immunogenic amount of an antigen. Suitably this is a vaccine in the form of an oil-in-water type emulsion, having oily particles dispersed in a continuous aqueous phase, for immunizing an animal, which vaccine comprises an immunogenic amount of an antigen; an emulsion-forming amount of a non-toxic tetra-polyol; optionally, an emulsion-forming amount of a non-toxic metabolizable oil; optionally an emulsion-stabilizing amount of a glycol ether-based surfactant; water or aqueous solution; and an immunopotentiating amount of a muramyldipeptide, preferably a derivative of formula I. A preferred subgenus is the vaccine which includes a tetra-polyol, especially where said tetra-polyol is Tetronic® 1501. A preferred subclass is the vaccine wherein said muramyldipeptide derivative of formula I is N-acetyl-muramyl-L-threonyl-D-isoglutamine. Another preferred subclass is the vaccine wherein said muramyldipeptide derivative of formula I is murabutide. A presently preferred embodiment is the vaccine which comprises: Tetronic® 1501 in an amount of 1-10%; squalane or squalene in an amount of 1-10%; Tween® 80 in an amount of about 0.2%; isotonic buffered saline; and 0.0001-10% N-acetylmuramyl-L-threonyl-D-isoglutamine.

Another aspect of the invention is a process for preparing the adjuvant or vaccine of the invention, which process comprises mixing together the aqueous phase and the emulsion-forming amount of the non-toxic tetra-polyol so as to form an emulsion.

Another aspect of the invention is a process for preparing an adjuvant of the invention, which process comprises: preparing a first mixture comprising a non-toxic tetra-polyol, optionally a non-toxic metabolizable oil, optionally a glycol ether- based surfactant, and water or aqueous solution; emulsifying said first mixture to produce an oil-in-water type emulsion, having oily particles dispersed in a continuous aqueous phase; and combining said emulsion with a muramyldipeptide derivative of formula I.

Another aspect of the invention is a kit for extemporaneous preparation of an adjuvant of the invention, which kit comprises:
a first container containing the emulsion of the tetra-polyol in the aqueous phase; and
a second container containing the glycopeptide.

Another aspect of the invention is a kit for extemporaneous preparation of a vaccine of the invention, which kit comprises:
a first container containing the emulsion of the tetra-polyol in the aqueous phase; and
a second container containing the antigen;
wherein the glycopeptide may be present in a third container, or in the first or second containers.

Another aspect of the invention is a kit for extemporaneous preparation of an adjuvant of the invention, which kit comprises: a first container containing an oil-in-water type emulsion, having oily particles dispersed in a continuous aqueous phase, where said emulsion comprises Tetronic® 1501,
squalane or squalene, optionally Tween® 80, and isotonic buffered saline; and a second container containing N-acetylmuramyl-L- threonyl-D-isoglutamine in powder form (preferably lyophilized) or in aqueous solution or suspension, where the concentrations of the components in each container are selected such that combination of the contents of both containers produces a formulation comprising Tetronic® 1501
in an amount of 1-30%, squalane or squalene in an amount of 1-30%, optionally Tween® 80 in an amount of about 0.2-5%, 0.0001-30% N-acetylmuramyl-L-threonyl-D-isoglutamine, and isotonic buffered saline. A preferred subgenus is the kit wherein Tetronic® 1501 is included.

Another aspect of the invention is a kit for extemporaneous preparation of a vaccine of the invention, which kit comprises: a first container containing an oil-in-water type emulsion, having oily particles dispersed in a continuous aqueous phase, where said emulsion comprises Tetronic® 1501, squalane or squalene, optionally Tween® 80, and isotonic buffered saline; and a second container containing N-acetylmuramyl-L-threonyl-D- isoglutamine in powder form (preferably lyophilized) or in aqueous solution or suspension and an immunogenic amount of an antigen; where the concentrations of the components in each container are selected such that combination of the contents of both containers produces a formulation comprising Tetronic® 1501 in an amount of 1-10%, squalane or squalene in an amount of 1-10%, optionally Tween® 80 in an amount of about 0.2%, 0.0001-10% N-acetylmuramyl-L-threonyl-D-isoglutamine, an immunogenic amount of an antigen, and isotonic buffered saline. Optionally, the antigen can be in a separate third container. A preferred subgenus is the kit wherein Tetronic® 1501 is included.

As noted, suitably in the kits of the invention the glycopeptide is present as a powder, preferably a lyophilized powder.

### DEFINITIONS

The term "alkyl" refers to a straight or branched radical comprised of 1 to 22 carbon atoms containing no unsaturation. Examples of alkyl are methyl, ethyl, propyl, butyl, tert-butyl, hexyl, octyl, decyl, dodecyl, eicosanyl, and the like. "Lower alkyl" refers to an alkyl radical of 1 to 7 carbon atoms, for example, methyl, ethyl, propyl, butyl, tert-butyl, hexyl, 3-methylhexyl, heptyl, and the like. "Cycloalkyl" refers to cyclopropyl, cyclobutyl, cyclopentyl, cyclohexyl, cycloheptyl, and the like.

The term "acyl" refers to radicals of the formula RCO-, where R is H or alkyl as defined above. "Lower acyl" refers to such radicals where R is H or lower alkyl. Examples of acyl include formyl, acetyl, propionyl, butyryl, pentanoyl, hexanoyl, eicosanoyl, and the like. Examples of lower acyl include formyl, acetyl, propionyl, butyryl, pentanoyl, hexanoyl, and the like.

The term "halo" as used herein refers to fluoro, chloro, bromo and iodo.

The term "alkoxy" refers to a radical of the form RO-, where R is lower alkyl or cycloalkyl as defined above.

The term "aryl" refers to aromatic radicals consisting entirely of carbon and hydrogen, containing from 6 to 12 carbon atoms. Examples of aryl groups are phenyl, naphthyl, and the like.

The term "pharmaceutically acceptable salts" refers to acid addition salts of the subject compounds which possess the desired pharmacological activity and which are neither biologically nor otherwise undesirable. These salts are formed with inorganic acids such as hydrochloric acid, hydrobromic acid, sulfuric acid, nitric acid or phosphoric acid; or organic acids such as acetic acid, propionic acid, glycolic acid, pyruvic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid and the like.

The term "treatment" as used herein covers any treatment of a disease in a bird or mammal, particularly a human, and includes:
(i) preventing the disease from occurring in a subject which may be predisposed to the disease but has not yet been diagnosed as having it;
(ii) inhibiting the disease, i.e., arresting its development; or
(iii) relieving the disease, i.e., causing regression of the disease. (It should be noted that vaccination may effect regression of a disease where the disease persists due to ineffective antigen recognition by the subject's immune system, where the vaccine effectively presents antigen.)

The term "optionally substituted" as applied to aryl radicals in the invention means that the radical may be unsubstituted or substituted with one to three halo, nitro, lower alkyl, or lower alkoxy groups. The optional substituents may be the same or different.

The term "muramyl dipeptide derivative" includes compounds of formula I: 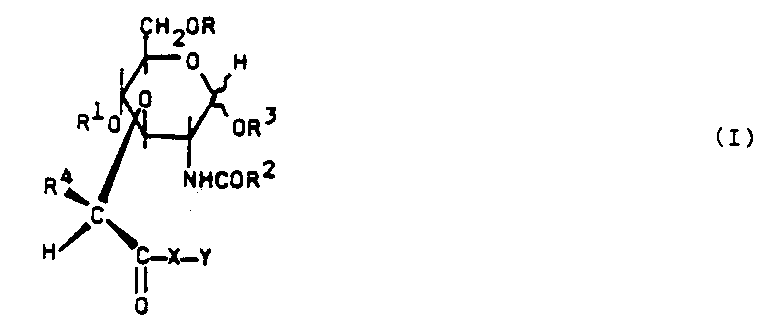 where R, R₁, R₂, R₃, and R₄ are each independently H, alkyl, acyl, or aryl optionally substituted with halo, nitro, or lower alkyl; X is one or several amino acids, and Y is D-glutamine, D-isoglutamine or D-isoasparagine, which may optionally be esterified or amidated. Preferred compounds are those of Formula 1 wherein R and R₁ are H or acyl of 1 to 22 carbon atoms; R₂ is methyl; R₃ is hydrogen; X is L-alanyl, L-α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl, and Y is D-glutamine or D-isoglutamine. The most preferred MDPs are: N-acetylmuramyl-L-α-aminobutyryl-D-isoglutamine; 6-O-stearoyl-N-acetylmuramyl-L-α-aminobutyryl-D-isoglutamine; N-acetylmuramyl-L-threonyl-D-isoglutamine; N-acetylmuramyl-L-valyl-D-isoglutamine; N-acetylmuramyl-L-alanyl-D-isoglutamine; N-acetyl-desmethylmuramyl-L-alanyl-D-isoglutamine; N-acetylmuramyl-L-alanyl-D-glutamine butyl ester (murabutide); N-acetylmuramyl-L-seryl-D-isoglutamine; and N-butyrylmuramyl-L-(α-aminobutyryl)-D-isoglutamine. Another useful MDP is N-acetyl-(n-butylmuramyl)-L-α-aminobutyryl-D-isoglutamine.

The term "immunopotentiating amount" refers to the amount of MDP derivative needed to effect an increase in antibody titer and/or cell mediated immunity when administered with an antigen in the formulation of the invention, as compared with the titer level observed in the absence of the MDP. As can be appreciated, each MDP may have an effective dose range that may differ from other MDPs. Thus, a single dose range cannot be prescribed which will have a precise fit for each possible glycopeptide within the scope of this invention. However, the immunopotentiating amount may easily be determined by one of ordinary skill in the art. As a general rule, the glycopeptide will preferably be present in an amount of between 0.001 and 2%. A more preferred amount is 0.005 to 1%.

The term "non-toxic metabolizable oil" refers to an oil of 6 to 30 carbon atoms including, but not limited to, alkanes, alkenes, alkynes, and their corresponding acids and alcohols, the ethers and esters thereof, and mixtures thereof. The oil may be any vegetable oil, fish oil, animal oil or synthetically prepared oil which can be metabolized in the body of the subject to which the adjuvant is administered, and which is not toxic to the organism. It is essential that the oil be metabolized by the animal or bird to which it is administered to avoid causing abscesses, granulomas or carcinomas. Nuts, seeds and grains are common sources of vegetable oils. Synthetic oils within the scope of this invention include "Neobee®" (available from PVO International, Inc., Chemical Specialities Division, 416 Division Street, Boongon, New Jersey) and others. Shark liver oil contains a branched, unsaturated oil known as squalene, 2,6,10,15,19,23-hexamethyl-2,6,10,14,18,22-tetracosahexene which is particularly preferred herein. Squalane, the saturated analog of squalene is also a particularly preferred oil. Fish oils, including squalene and squalane, are readily available from commercial sources or may be obtained by methods known in the art.

An "emulsion-forming amount" of a non-toxic metabolizable oil is that amount which will form an emulsion in the presence of the tetra-polyol. The oil component of these adjuvants and vaccine formulations will usually be present in an amount between 1 to 30%, but preferably in an amount of 1 to 10%. It is most preferred to use about a 5% concentration of oil.

The aqueous portion of these adjuvant compositions is preferably buffered isoosmotic saline. Because these compositions are intended for parenteral administration, it is preferred to formulate these solutions so that the tonicity is essentially the same as normal physiological fluids in order to prevent post-administration swelling or rapid absorption of the composition due to differential ion concentrations between the composition and physiological fluids. It is also preferred to buffer the saline in order to maintain a pH compatible with normal physiological conditions. Also, in certain instances, it may be necessary to maintain the pH at a particular level in order to insure the stability of certain composition components, such as the glycopeptides. Any physiologically acceptable buffer may be used herein, but it has been found that it is most convenient to use a phosphate buffer. Any other acceptable buffer such as acetate, Tris, bicarbonate, carbonate, and the like can be used as a substitute for a phosphate buffer. It is preferred to use phosphate buffered saline, or saline buffered with a mixture of phosphate and acetate.

The term "antigen" refers to any substance, usually a protein or glycoprotein, lipoprotein, saccharide, polysaccharide or lipopolysaccharide, which upon administration stimulates the formation of specific antibodies and reacts specifically in vivo or in vitro with a homologous antibody. Moreover, it stimulates the proliferation of T-lymphocytes with receptors for the antigen, and can react with the lymphocytes to initiate the series of responses designated cell-mediated immunity.

The term "antigen" as used herein also includes combinations of haptens with a carrier. A hapten is a portion of an antigenic molecule or antigenic complex that determines its immunological specificity, but is not sufficient to stimulate an immune response in the absence of a carrier. Commonly, a hapten is a relatively small peptide or polysaccharide and may be a fragment of a naturally occurring antigen. In artificial antigens, it may be a low molecular weight substance such as, for example, an arsanilic acid derivative. A hapten will react specifically in vivo and in vitro with homologous antibodies or T-lymphocytes. Haptens are typically attached to a large carrier molecule such as bovine serum albumin (BSA) or keyhole limpet hemocyanin (KLH) by either covalent or non-covalent binding before formulation as a vaccine. For example, a common artificial antigen used to test vaccines and adjuvants consists of 2,4-dinitrophenol (DNP) covalently bound to BSA. Suitable antigens for use in this invention include hepatitis B (surface) antigen, and influenza (for example, A or B) antigen. Also, antigens for AIDS and herpes.

The term "immunogenic amount" of an antigen refers to an amount of antigen sufficient to stimulate a useful immune response, when administered with an adjuvant of the invention. The amount of antigen necessary to provide an immunogenic amount is readily determined by one of ordinary skill in the art, e.g., by preparing a series of vaccines of the invention with varying concentrations of antigen, administering the vaccines to suitable laboratory animals (e.g., Guinea pigs), and assaying the resulting immune response by measuring serum antibody titer, antigen-induced swelling in the skin, and the like.

The term "tetra-polyol" as used herein refers to N,N,N',N'-tetra(polyoxypropylene-polyoxyethylene)-1,2-diaminoethane block polymers. These compounds may be prepared by the process disclosed in U.S. Patent No. 2,979,528, or may be obtained commercially from BASF-Wyandotte under the trademark Tetronic®.

Tetronic® polyols are designated with a three or four digit number which indicates the average molecular weight of the polyoxypropylene (POP) portion and the percentage of the total molecular weight contributed by the polyoxyethylene (POE) portion of the molecule. The first one or two non-zero digits indicate the average molecular weight of the POP section, ranging from 501-1000 for Tetronic® 304 to 6500-7000 for Tetronic® 1501. The last digit indicates the percentage of POE in 10% increments, ranging from 10% for Tetronic® 1501 to 80% for Tetronic® 1508. The characteristics of these compounds are determined by the molecular weight of the POP portion and the amount of POE in the product. Preferred tetra-polyols in the practice of the invention are relatively insoluble in water at 25°C and have low HLB values. The HLB value should preferably be lower than about 5.0. Presently preferred tetra-polyols are Tetronic® 1501, Tetronic® 1301, Tetronic® 1101, and Tetronic® 1502, particularly Tetronic® 1501 and Tetronic® 1301, especially Tetronic® 1501. Other appropriate tetra-polyols with the necessary properties may be prepared using the methods disclosed in U.S. Patent No. 2,979,528, and are to be considered equivalents within the scope of this invention. For example, one could prepare a tetra-polyol with a POP molecular weight of 8,000 and a POE content of 8%. The properties necessary are (i) low HLB value (≤ 5.0, preferably ≤ 2.0); (ii) little or no aqueous solubility; (iii) forms stable emulsions with the addition of a glycol ether-based surfactant; and (iv) lack of toxicity.

An "emulsion-forming amount" of tetra-polyol is that quantity which will form micelles or an emulsion. For the purposes of the invention this is an amount between 0.2% and 49% by volume. A more preferred amount is from 0.2% to 20%, and about 1-5% is even more preferred. A concentration of 1-2.5% is presently most preferred.

The term "surfactant" refers to non-toxic surface active agents capable of stabilizing the emulsion. There are a substantial number of emulsifying and suspending agents generally used in the pharmaceutical sciences. These include naturally derived materials such as gums, vegetable protein, alginates, cellulose derivatives, phospholipids (whether natural or synthetic), and the like. Certain polymers having a hydrophilic substituent on the polymer backbone have surfactant activity, for example, povidone, polyvinyl alcohol, and glycol ether-based compounds. Compounds derived from long chain fatty acids are a third substantial group of emulsifying and suspending agents usable in this invention. Though any of the foregoing surfactants can be used so long as they are non-toxic, glycol ether-based surfactants are preferred. Preferred surfactants are non-ionic. These include polyethylene glycols (especially PEG 200, 300, 400, 600 and 900), Span®, Arlacel®, Tween®, Myrj®, Brij® (all available from ICI, America's Inc., Wilmington, Delaware), polyoxyethylene, polyol fatty acid esters, polyoxyethylene ether, polyoxypropylene fatty ethers, bee's wax derivatives containing polyoxyethylene, polyoxyethylene lanolin derivatives, polyoxyethylene fatty glycerides, glycerol fatty acid esters or other polyoxyethylene acid alcohol or ether derivatives of long-chain fatty acids of 12-21 carbon atoms. The presently preferred surfactant is Tween® 80 (otherwise known as polysorbate 80 for polyoxyethylene 20 sorbitan monooleate), although it should be understood that any of the above-mentioned surfactants would be suitable after lack of toxicity is demonstrated.

An "emulsion-stabilizing amount of a glycol ether-based surfactant" is usually effected by having the surfactant present in an amount of 0.05 to 5%. An amount of 0.2% to 1% is preferred.

### PREPARATION

The components of the adjuvant of the invention may be obtained through commercial sources, or may be prepared by one of ordinary skill in the art.

The tetra-polyols may be prepared by the process disclosed in U.S. Patent No. 2,979,528, or may be obtained commercially from BASF-Wyandotte under the trademark Tetronic®.

The glycol-ether based surfactants PEG 200, 300, 400, 600 and 900, Span®, Arlacel®, Tween®, Myrj®, Brij®, and the like are readily available commercially from ICI, America's Inc., Wilmington, Delaware, and others.

The non-toxic metabolizable oils are available from a variety of sources: e.g., squalane and squalene are available from Aldrich Chemical Co.

The MDPs may be obtained commercially from sources such as Sigma Chemical Co., or prepared following the processes disclosed in Audibert et al., U.S. Pat. No. 4,158,052; Audibert et al., U.S. Pat. No. 4,220,637; Audibert et al., U.S. Pat. No. 4,323,559; Baschang et al., U.S. Pat. No. 4,323,560; Baschang et al., U.S. Pat. No. 4,409,209; Baschang et al., U.S. Pat. No. 4,423,038; Derrien et al., U.S. Pat. No. 4,185,089; Hartmann et al., U.S. Pat. No. 4,406,889; Jones et al., U.S. Pat. No. 4,082,735; Jones et al., U.S. Pat. No. 4,082,736; Le Francier et al., U.S. Pat. No. 4,427,659; Le Francier et al., U.S. Pat. No. 4,461,761; Yamamura et al., U.S. Pat. No. 4,314,998; Yamamura et al., U.S. Pat. No. 4,101,536; and Yamamura et al., U.S. Pat. No. 4,369,178, all incorporated herein by reference.

The adjuvant is prepared by emulsification, using a mixer. If the adjuvant is to be prepared on a laboratory scale using a tetra-polyol for immediate use, it may be mixed simply by hand. For example, Tween® 80 and buffered saline are added to squalane and Tetronic® 1501 in a test tube at 2X concentration, and the combination mixed using a vortex mixer to form an emulsion. To this is added a 2X solution of antigen and MDP in buffered saline to form the completed vaccine. It is more preferred to use a high-shear mixer such as a Greerco Homogenizer Mixer to form a smoother, more homogenous emulsion.

Preferably, the adjuvant emulsion is "microfluidized" prior to adding the antigen.

Normally, the MDP will be added after the emulsification step. If the emulsion is to be stored prior to the addition of the MDP and the antigen, this may be done with refrigeration and/or under nitrogen.

The resulting emulsion may be assayed in a variety of ways well known in the art. The emulsion stability may be measured by allowing the emulsion to stand at room temperature, and under refrigeration, followed by observation for separation into phases. This assay may be accelerated by centrifuging the emulsion, e.g., for two hours at 4500g.

Particle size may be determined by, for example, optical microscopy, transmission electron microscopy, and laser light-scattering techniques, preferably using laser photon correlation spectroscopy (PCS). PCS analysis may be performed using, for example, a Nicomp Model 200 laser particle sizer, with a model TC-100 computing autocorrelator.

Biological activity may be assayed using standard laboratory techniques, e.g., by vaccinating a standard laboratory animal (e.g., a Guinea pig) with a standard antigen (e.g., BSA or DNP-BSA) using a test adjuvant formulation. After allowance of time for boosting the vaccination, and time for immunization to occur, the animal is challenged with the standard antigen and the results measured. The response may be quantified by any measure accepted in the art for measuring immune responses, e.g., in terms of serum antibody titer against the standard antigen (for humoral immunity) and skin test reaction (for cell-mediated immunity).

### ADMINISTRATION

It will be apparent to one of ordinary skill in the art that the precise amounts of MDP derivative and antigen needed to produce a given effect will vary with the particular compounds and antigens, and with the size, age, and condition of the subject to be treated. Thus, it is impossible to state exactly the amounts needed: however, these amounts can easily be determined using methods known to those of ordinary skill in the art.

The adjuvants and vaccines of the invention are generally administered by injection, particularly intramuscular injection, preferably into a large muscle.

In general, an initial vaccination is administered using the desired antigen and the formulation of the invention. The vaccination is "boosted" several weeks later (usually 2-6 weeks, for example, 4-6 weeks) using a vaccine of the invention with or without (preferably with) the MDP component. Generally, 1-2 mL of a vaccine (such as are described in the Examples below) is administered to a human subject in the practice of the invention.

The following examples are presented as an aid to those of ordinary skill in the art, and are not to be considered as a limitation of the invention in any way.

### EXAMPLE 1

### (Immunogenicity)

### (A) Preparation of Formulations:

Adjuvant formulations were prepared as follows for assay of biological activity. Each emulsion was prepared at 2X concentration prior to combination with a 2X solution of antigen.
Formulation 1 (from Allison, U.S. 4,606,918): 5.0% Pluronic® L121, 10% squalane, 0.4% Tween® 80, qs phosphate buffered saline (pH 7.4); the components were added to a test tube and vortex-mixed until a milky emulsion was obtained. This formulation was prepared immediately prior to administration.
Formulation 2 (tetra-polyol formulation of the invention): 5.0% Tetronic® 1501, 10% squalane, 0.4% Tween® 80, qs phosphate buffered saline (pH 7.4); the components were added to a test tube and vortex-mixed until a milky emulsion was obtained.

To each formulation was then added solid N-acetyl-muramyl-L-threonyl-D-isoglutamine (Thr-MDP) to a concentration of 500 µg/mL, to form the complete adjuvant "concentrate." The concentrate was then mixed with a 2X concentration solution of antigen (ovalbumin in saline, 1 mg/mL) to form a test vaccine.

### (B) Bioactivity:

Each test vaccine (0.2 mL) was administered to 8 female Guinea pigs. At four weeks following administration, each animal was boosted with the same test vaccine (but without the Thr-MDP). Antibody titer was measured from serum samples collected at weeks 4 and 6 after initial administration. At 6 weeks, each animal received ovalbumin intradermally, and the diameter of the erythema, and the infiltration rating were determined after 24 hours, as an indication of cell-mediated immunity.

The results are reported in Tables 1 and 2. Each entry represents the mean obtained from 8 animals. Antibody titers were determined by hemagglutination. Infiltration was scored visually, on a 1-3 scale (1 being the weakest response, and 3 being a very obvious swelling at the skin test site).

**TABLE 1**

| Antibody Titer Results | | |
|---|---|---|
| Formulation | 4 weeks | 6 weeks |
| 1 (control POP-POE) | 2.25 ± .38 | 6.13 ± .30 |
| 2 (tetra-polyol) | 4.00 ± .38 | 8.13 ± .30 |

**TABLE 2**

| Cell-Mediated Immunity Results | | |
|---|---|---|
| Formulation | Diameter (mm) | Infiltration |
| 1 (control POP-POE) | 16.06 | 1.75 |
| 2 (tetra-polyol) | 19.13 | 1.94 |

The results demonstrate that the tetra-polyol adjuvants are significantly more effective for increasing the immunogenicity of antigens.

### EXAMPLE 2

### (Formulations)

Exemplary adjuvant formulations were prepared as follows:
(A) Tetronic®/Thr-MDP:

| | |
|---|---|
| Tetronic® 1501 | 2.5 g |
| Squalane | 5.0 g |
| Tween® 80 | 0.2 g |
| Thr-MDP | 250.0 mg |
| Phosphate buffered saline qs to | 100.0 mL |

The Tetronic® 1501, squalane, and Tween® 80 are placed in an appropriate vessel with 85 mL of phosphate buffered saline (PBS) and are mixed with a mechanical mixer (Greerco Homogenizer-Mixer, model #1L-79, Greerco Corp., Hudson, New Hampshire) at about 4750 rpm for about 30-60 minutes. Then, the Thr-MDP (N-acetylmuramyl-L-threonyl-D-isoglutamine) and remaining 15 mL of PBS are stirred in, producing an adjuvant formulation of the invention.
(B) Similarly, proceeding as in part (A) above but substituting N-acetylmuramyl-L-α-aminobutyryl-D-isoglutamine (Abu-MDP), 6-O-stearoyl-N-acetylmuramyl-L-α-aminobutyryl-D-isoglutamine (Abu-MDP stearate), N-acetylmuramyl-L-valyl-D-isoglutamine (Val-MDP), N-acetylmuramyl-L-alanyl-D-isoglutamine (MDP), N-acetyl-desmethylmuramyl-L-alanyl-D-isoglutamine (desMe-MDP), N-acetylmuramyl-L-alanyl-D-glutamine butyl ester (murabutide), n-butyrylmuramyl-L-(α-aminobutyryl)-D-isoglutamine, and N-acetylmuramyl-L-seryl-D-isoglutamine (Ser-MDP), for the Thr-MDP, the corresponding adjuvant formulations are prepared.
(c) Tetronic®/murabutide:

| | |
|---|---|
| Tetronic® 1501 | 2.0 g |
| Squalane | 6.0 g |
| Brij® 80 | 0.3 g |
| murabutide | 300.0 mg |
| Phosphate buffered saline qs to | 100.0 mL |

The formulation is prepared as described in part A above.
(D) Similarly, proceeding as described in parts A-C above, but substituting Tetronic® 1301 for Tetronic® 1501, the corresponding adjuvants are prepared.
(E) Vaccines: Vaccines of the invention are prepared by adding an appropriate amount of antigen to any of the formulations described above. Suitable antigens include antigens for hepatitis B, influenza (for example, A or B), AIDS and herpes. The vaccine may contain more than one antigen if desired, for example, antigens for diphtheria, pertussis, and tuberculosis may be coadministered in a single formulation.

For ease of preparation, a small portion of the PBS used may be withheld from the adjuvant preparation, e.g., one may prepare the adjuvants described above using 90 mL rather than 100 mL, and use the withheld PBS to dissolve/suspend the antigen(s). The antigen/PBS solution is then mixed with the (slightly) concentrated emulsion to prepare the final vaccine. Alternatively, and more preferably, an adjuvant emulsion (without MDP) of two times concentration is mixed with an antigen/MDP solution of two times concentration.

## Claims

1. An adjuvant in the form of an emulsion having oily particles dispersed in a continuous aqueous phase, for potentiating the immunogenicity of an antigen, which adjuvant comprises:
an emulsion-forming amount of a non-toxic N,N,N',N'-tetra(polyoxypropylene-polyoxyethylene)-1,2-diaminoethane block polymer ("tetra-polyol"); and
an immunopotentiating amount of a glycopeptide.

2. An adjuvant according to Claim 1 in the form of an emulsion having oily particles dispersed in a continuous aqueous phase, for potentiating the immunogenicity of an antigen, which adjuvant comprises:
an emulsion-forming amount of a non-toxic tetra-polyol;
optionally, an emulsion-forming amount of a non-toxic metabolizable oil;
an emulsion-stabilizing amount of a glycol ether-based surfactant;
water or aqueous solution; and
an immunopotentiating amount of a muramyldipeptide derivative of formula I 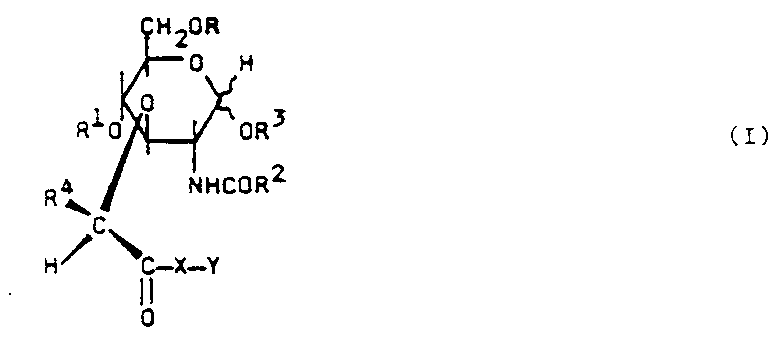 and the pharmaceutically acceptable salts thereof, wherein
R and R₁ are each independently H or acyl of 1 to 22 carbon atoms;
R₂ is alkyl or aryl, optionally substituted with halo, nitro, or lower alkyl;
R₃ is H, alkyl, or aryl;
R₄ is H or lower alkyl;
X is L-alanyl, L- α-aminobutyryl, L-arginyl, L-asparginyl, L-aspartyl, L-cysteinyl, L-glutaminyl, L-glutamyl, glycyl, L-histidyl, L-hydroxyprolyl, L-isoleucyl, L-leucyl, L-lysyl, L-methionyl, L-ornithinyl, L-phenylalanyl, L-prolyl, L-seryl, L-threonyl, L-tyrosyl, L-tryptophanyl, or L-valyl; and
Y is D-glutamine, D-isoglutamine or D-isoasparagine.

3. The adjuvant of Claim 1 or 2 wherein said tetra-polyol is Tetronic® 1501 which has an average molecular weight of the polyoxypropylene (POP) section of 6500-7000, and a percentage of polyoxyethylene (POE) of 10%.

4. The adjuvant of Claim 3 which includes a non-toxic metabolizable oil, wherein said oil is squalene or squalane.

5. The adjuvant of Claim 3 or 4 wherein said glycol ether-based surfactant is Tween® 80.

6. The adjuvant of Claim 3, 4 or 5 wherein said water or aqueous solution comprises isotonic buffered saline.

7. The adjuvant of any one of Claims 2-6 wherein said muramyl- dipeptide derivative of formula I is:
N-acetylmuramyl-L-threonyl-D-isoglutamine,
N-acetylmuramyl-L- α-aminobutyryl-D-isoglutamine,
6-O-stearoyl-N-acetylmuramyl-L- α-aminobutyryl-D-isoglutamine,
N-acetylmuramyl-L-valyl-D-isoglutamine,
N-acetylmuramyl-L-alanyl-D-isoglutamine,
N-acetyl-desmethylmuramyl-L-alanyl-D-isoglutamine,
N-acetylmuramyl-L-alanyl-D-glutamine butyl ester,
N-acetylmuramyl-L-seryl-D-isoglutamine, or
N-butyrylmuramyl-L- α-aminobutyryl-D-isoglutamine.

8. An adjuvant according to any one of the preceding claims in the form of an oil-in-water type emulsion, having oily particles dispersed in a continuous aqueous phase, for potentiating the immunogenicity of an antigen, which adjuvant comprises:
a non-toxic tetra-polyol in an amount of 0.2 to 49%;
a non-toxic metabolizable oil in an amount of 0-15%;
a glycol ether-based surfactant in an amount of 0.05-5%;
water or aqueous solution; and
0.0001-10% a muramyldipeptide derivative of formula I.

9. An adjuvant according to Claim 8 in the form of an oil-in-water type emulsion, having oily particles dispersed in a continuous aqueous phase, for potentiating the immunogenicity of an antigen, which adjuvant comprises:
Tetronic® 1501 in an amount of 1-10%;
squalane or squalene in an amount of 1-10%;
Tween® 80 in an amount of about 0.2%;
isotonic buffered saline; and
0.0001-10% N-acetylmuramyl-L-threonyl-D-isoglutamine.

10. A vaccine in the form of an oil-in-water type emulsion, having oily particles dispersed in a continuous aqueous phase, for immunizing an animal, which vaccine comprises:
an immunogenic amount of an antigen; and
an adjuvant according to any one of the Claims 1-9.

11. A process for preparing the adjuvant according to any one of claims 1-9, which process comprises mixing together the aqueous phase and the emulsion-forming amount of the non-toxic tetra-polyol so as to form an emulsion.

12. A process according to Claim 11 wherein the glycopeptide is added to the mixture after the emulsion is formed.

13. A process for preparing the vaccine according to Claim 10, which process comprises mixing the antigen with the adjuvant.

14. A kit for extemporaneous preparation of an adjuvant according to any one of Claims 1-9 which kit comprises:
a first container containing the emulsion of the tetra-polyol in the aqueous phase; and
a second container containing the glycopeptide.

15. A kit according to Claim 14 for extemporaneous preparation of an adjuvant of the invention, which kit comprises:
a first container containing an oil-in-water type emulsion, having oily particles dispersed in a continuous aqueous phase, where said emulsion comprises Tetronic® 1501, squalane or squalene, Tween® 80, and isotonic buffered saline, and
a second container containing N-acetylmuramyl-L-threonyl-D-isoglutamine;
where the concentrations of the components in each container are selected such that combination of the contents of both containers produces a formulation comprising Tetronic® 1501 in an amount of 1-30%, squalane or squalene in an amount of 1-30%, Tween® 80 in an amount of about 0.2-5%, 0.0001-30% N-acetylmuramyl-L-threonyl-D-isoglutamine, and isotonic buffered saline.

16. A kit for extemporaneous preparation of a vaccine according to Claim 10, which kit comprises:
a first container containing the emulsion of the tetra-polyol in the aqueous phase; and
a second container containing the antigen;
wherein the glycopeptide may be present in a third container, or in the first or second containers.

17. A kit according to Claim 16 for extemporaneous preparation of a vaccine of the invention, which kit comprises:
a first container containing an oil-in-water type emulsion, having oily particles dispersed in a continuous aqueous phase, where said emulsion comprises Tetronic® 1501, squalane or squalene, Tween® 80, and isotonic buffered saline; and
a second container containing N-acetylmuramyl-L-threonyl-D-isoglutamine and an immunogenic amount of an antigen;
where the concentrations of the components in each container are selected such that combination of the contents of both containers produces a formulation comprising Tetronic® 1501 in an amount of 1-10%, squalane or squalene in an amount of 1-10%, Tween® 80 in an amount of about 0.2%, 0.0001-10% N-acetylmuramyl-L-threonyl-D-isoglutamine, an immunogenic amount of an antigen, and isotonic buffered saline.

## Patentansprüche

1. Adjuvans in Form einer Emulsion mit in einer kontinuierlichen wäßrigen Phase dispergierten Öl-Teilchen zur Potenzierung der Immunogenität eines Antigens, wobei das Adjuvans umfaßt:
eine Emulsions-bildende Menge eines nicht-toxischen N,N,N',N'-Tetra (polyoxypropylen-polyoxyethylen)-1,2-diaminoethan-Block-Polymers ("Tetra-Polyols"); und
eine immunpotenzierende Menge eines Glycopeptids.

2. Adjuvans nach Anspruch 1 in Form einer Emulsion mit in einer kontinuierlichen wäßrigen Phase dispergierten Öl-Teilchen zur Potenzierung der Immunogenität eines Antigens, wobei das Adjuvans umfaßt:
eine Emulsions-bildende Menge eines nicht-toxischen Tetra-Polyols;
gegebenenfalls eine Emulsions-bildende Menge eines nicht-toxischen metabolisierbaren Öls;
eine Emulsions-stabilisierende Menge eines Tensids auf Glycolether-Basis;
Wasser oder wäßrige Lösung; und
eine immunpotenzierende Menge eines Muramyldipeptid-Derivats der Formel (I) 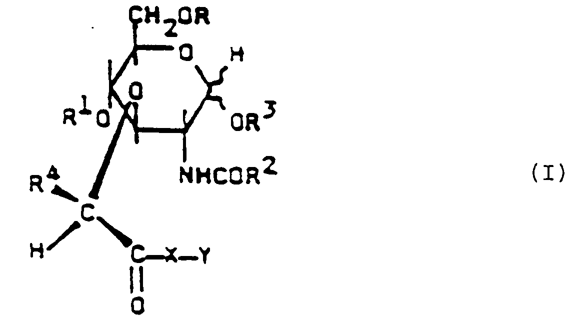 und der pharmazeutisch annehmbaren Salze davon,
worin
R und R₁ jeweils unabhängig für H oder Acyl mit 1 bis 22 Kohlenstoffatomen stehen;
R₂ Alkyl oder Aryl, gegebenenfalls mit Halogen, Nitro oder Niederalkyl substituiert, darstellt;
R₃ H, Alkyl oder Aryl repräsentiert;
R₄ H oder Niederalkyl bedeutet;
X für L-Alanyl, L-α-Aminobutyryl, L-Arginyl, L-Asparginyl, L-Aspartyl, L-Cysteinyl, L-Glutaminyl, L-Glutamyl, Glycyl, L-Histidyl, L-Hydroxyprolyl, L-Isoleucyl, L-Leucyl, L-Lysyl, L-Methionyl, L-Ornithinyl, L-Phenylalanyl, L-Prolyl, L-Seryl, L-Threonyl, L-Tyrosyl, L-Tryptophanyl oder L-Valyl steht; und
Y D-Glutamin, D-Isoglutamin oder D-Isoasparagin ist.

3. Adjuvans nach Anspruch 1 oder 2, in welchem das Tetra-Polyol Tetronic® 1501 ist, welches ein durchschnittliches Molekulargewicht des Polyoxypropylen (POP)-Abschnitts von 6500 - 7000 und einen Prozentsatz an Polyoxyethylen (POE) von 10% aufweist.

4. Adjuvans nach Anspruch 3, welches ein nicht-toxisches metabolisierbares Öl einschließt, wobei das Öl Squalen oder Squalan ist.

5. Adjuvans nach Anspruch 3 oder 4, in welchem das Tensid auf Glycolether-Basis Tween® 80 ist.

6. Adjuvans nach Anspruch 3, 4 oder 5, in welchem das Wasser oder wie wäßrige Lösung isotone gepufferte Salzlösung umfaßt.

7. Adjuvans nach irgendeinem der Ansprüche 2 - 6, in welchem es sich bei dem Muramyl-Dipeptid-Derivat der Formel I handelt um:
N-Acetylmuramyl-L-threonyl-D-isoglutamin,
N-Acetylmuramyl-L-α-aminobutyryl-D-isoglutamin,
6-O-Stearoyl-N-acetylmuramyl-L-α-aminobutyryl-D-isoglutamin,
N-Acetylmuramyl-L-valyl-D-isoglutamin,
N-Acetylmuramyl-L-alanyl-D-isoglutamin,
N-Acetyl-desmethylmuramyl-L-alanyl-D-isoglutamin,
N-Acetylmuramyl-L-alanyl-D-glutaminbutylester,
N-Acetylmuramyl-L-seryl-D-isoglutamin oder
N-Butyrylmuramyl-L-α-aminobutyryl-D-isoglutamin.

8. Adjuvans nach irgendeinem der vorangehenden Ansprüche in Form einer Emulsion vom Öl-in-Wasser-Typ, die in einer kontinuierlichen wäßrigen Phase dispergierte Öl-Teilchen aufweist, zur Potenzierung der Immunogenität eines Antigens, wobei das Adjuvans umfaßt:
ein nicht-toxisches Tetra-Polyol in einer Menge von 0,2 bis 49%;
ein nicht-toxisches metabolisierbares Öl in einer Menge von 0 - 15%;
ein Tensid auf Glycolether-Basis in einer Menge von 0,05 - 5%;
Wasser oder eine wäßrige Lösung; und
0,0001 - 10% eines Muramyldipeptid-Derivats der Formel I.

9. Adjuvans nach Anspruch 8 in Form einer Emulsion vom Öl-in-Wasser-Typ, die in einer kontinuierlichen wäßrigen Phasedispergierte Öl-Teilchenaufweist, zur Potenzierung der Immunogenität eines Antigens, wobei das Adjuvans umfaßt:
Tetronic® 1501 in einer Menge von 1 - 10%;
Squalan oder Squalen in einer Menge von 1 - 10%;
Tween® 80 in einer Menge von etwa 0,2%;
isotone gepufferte Salzlösung; und
0,0001 - 10% N-Acetylmuramyl-L-threonyl-D-isoglutamin.

10. Impfstoff in Form einer Emulsion vom Öl-in-Wasser-Typ, die in einer kontinuierlichen wäßrigen Phase dispergierte Öl-Teilchen aufweist, zur Immunisierung eines Tieres, wobei der Impfstoff umfaßt:
eine immunogene Menge eines Antigens; und
ein Adjuvans nach irgendeinem der Ansprüche 1 - 9.

11. Verfahren zur Herstellung des Adjuvans nach irgendeinem der Ansprüche 1 - 9, umfassend das Zusammenmischen der wäßrigen Phase und der Emulsions-bildenden Menge des nicht-toxischen Tetra-Polyols, um eine Emulsion zu bilden.

12. Verfahren nach Anspruch 11, in welchem das Glycopeptid der Mischung nach Bildung der Emulsion zugegeben wird.

13. Verfahren zur Herstellung des Impfstoffs nach Anspruch 10, umfassend das Mischen des Antigens mit dem Adjuvans

14. Satz zur extemporalen Herstellung eines Adjuvans nach irgendeinem der Ansprüche 1 - 9, umfassend:
einen ersten Behälter, der die Emulsion des Tetra-Polyols in der wäßrigen Phase enthält; und
einen zweiten Behälter, der das Glycopeptid enthält.

15. Satz nach Anspruch 14 zur extemporalen Herstellung eines Adjuvans der Erfindung, umfassend:
einen ersten Behälter, der eine Emulsion vom Öl-in-Wasser-Typ enthält, die in einer kontinuierlichen wäßrigen Phase dispergierte Öl-Teilchen aufweist, wobei die Emulsion Tetronic® 1501, Squalan oder Squalen, Tween® 80 und isotone gepufferte Salzlösung enthält, und
einen zweiten Behälter, der N-Acetylmuramyl-L-threonyl-D-isoglutamin enthält;
wobei die Konzentrationen der Komponenten in jedem Behälter so gewählt sind, daß die Vereingung des Inhalts beider Behälter eine Formulierung liefert, die Tetronic® 1501 in einer Menge von 1 - 30%, Squalan oder Squalen in einer Menge von 1 - 30%, Tween® 80 in einer Menge von etwa 0,2 - 5%, 0,0001 - 30% N-Acetylmuramyl-L-threonyl-D-isoglutamin und isotone gepufferte Salzlösung umfaßt.

16. Satz zur extemporalen Herstellung eines Impfstoffs nach Anspruch 10, umfassend:
einen ersten Behälter, der die Emulsion des Tetra-Polyols in der wäßrigen Phase enthält; und
einen zweiten Behälter, der das Antigen enthält;
wobei das Glycopeptid in einem dritten Behälter oder in dem ersten oder zweiten Behälter anwesend sein kann.

17. Satz nach Anspruch 16 zur extemporalen Herstellung eines Impfstoffs der Erfindung, umfassend:
einen ersten Behälter, der eine Emulsion vom Öl-in-Wasser-Typ enthält, die in einer kontinuierlichen wäßrigen Phase dispergierte Öl-Teilchen aufweist, wobei die Emulsion Tetronic® 1501, Squalan oder Squalen, Tween® 80 und isotone gepufferte Salzlösung umfaßt; und
einen zweiten Behälter, der N-Acetylmuramyl-L-threonyl-D-isoglutamin und eine immunogene Menge eines Antigens enthält;
wobei die Konzentrationen der Komponenten in jedem Behälter so gewählt sind, daß die Vereinigung des Inhalts der beiden Behälter eine Formulierung liefert, die Tetronic® 1501 in einer Menge von 1 - 10%, Squalan oder Squalen in einer Menge von 1 - 10%, Tween® 80 in einer Menge von etwa 0,2%, 0,0001 - 10% N-Acetylmuramyl-L-threonyl-D-isoglutamin, eine immunogene Menge eines Antigens und isotone gepufferte Salzlösung umfaßt.

## Revendications

1. Adjuvant sous forme d'émulsion contenant des particules huileuses en dispersion dans une phase aqueuse continue, destiné à potentialiser l'immunogénicité d'un antigène, lequel adjuvant comprend :
une quantité émulsifiante d'un polymère séquencé non toxique de type N,N,N',N'-tétra(polyoxypropylène-polyoxyéthylène)-1, 2-diaminoéthane ("tétra-polyol"); et
une quantité suffisante d'un glycopeptide pour potentialiser la réponse immune.

2. Adjuvant selon la revendication 1, sous forme d'émulsion contenant des particules huileuses en dispersion dans une phase aqueuse continue, destiné à potentialiser l'immunogenicité d'un antigène, lequel adjuvant comprend :
une quantité émulsifiante d'un tétra-polyol non toxique;
le cas échéant, une quantité émulsifiante d'une huile métabolisable non toxique;
une quantité stabilisatrice d'émulsion d'un tensioactif à base d'un éther de glycol ;
de l'eau ou une solution aqueuse; et
une quantité suffisante pour potentialiser la réponse immune d'un dérivé de muramyldipeptide de formule I 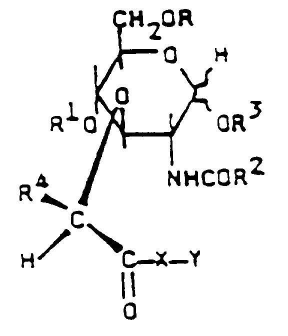
ou d'un de ses sels pharmaceutiquement acceptables, formule dans laquelle
R et R₁ représentent indépendamment chacun H ou un groupe acyle de 1 à 22 atomes de carbone;
R₂ est un groupe alkyle ou aryle, éventuellement substitué par un groupe halogéno, nitro, ou alkyle inférieur;
R₃ est H, un groupe alkyle, ou aryle;
R₄ est H ou un groupe alkyle inférieur;
X est un résidu L-alanyle, L-α-aminobutyryle, L-arginyle, L-asparaginyle, L-aspartyle, L-cystéinyle, L-glutaminyle, L-glutamyle, glycyle, L-histidyle, L-hydroxyprolyle, L-isoleucyle, L-leucyle, L-lysyle, L-méthionyle, L-ornithinyle, L-phénylalanyle, L-prolyle, L-séryle, L-thréonyle, L-tyrosyle, L-tryptophanyle, ou L-valyle; et
Y est un résidu D-glutamine, D-isoglutamine ou D-isoasparagine.

3. Adjuvant selon la revendication 1 ou 2, dans lequel ledit tétra-polyol consiste en Tetronic® 1501 dont la séquence de polyoxypropylène (POP) a un poids moléculaire moyen de 6500 à 7000, et dont la teneur en pourcentage de polyoxyéthylène (POE) est de 10%.

4. Adjuvant selon la revendication 3, qui comprend une huile métabolisable non toxique, dans lequel ladite huile est le squalène ou le squalane.

5. Adjuvant selon la revendication 3 ou 4, dans lequel ledit tensio-actif à base d'éther de glycol est le Tween 80.

6. Adjuvant selon la revendication 3, 4 ou 5, dans lequel ladite eau ou solution aqueuse comprend du tampon salin isotonique.

7. Adjuvant selon l'une quelconque des revendications 2 à 6, dans lequel le dérivé de muramyldipeptide de formule I est :
la N-acétylmuramyl-L-thréonyl-D-isoglutamine,
la N-acétylmuramyl-L-α-aminobutyryl-D-isoglutamine,
la 6-O-stéaroyl-N-acétylmuramyl-L-α-aminobutyryl-D-isoglutamine,
la N-acétylmuramyl-L-valyl-D-isoglutamine,
la N-acétylmuramyl-L-alanyl-D-isoglutamine,
la N-acétyl-desméthylmuramyl-L-alanyl-D-isoglutamine,
l'ester butylique de N-acétylmuramyl-L-alanyl-D-glutamine,
la N-acétylmuramyl-L-séryl-D-isoglutamine, ou
la N-butyrylmuramyl-L-α-aminobutyryl-D-isoglutamine.

8. Adjuvant selon l'une quelconque des revendications précédentes, sous forme d'une émulsion de type huile-dans-eau, contenant des particules huileuses en dispersion dans une phase aqueuse continue, destiné à potentialiser l'immunogénicité d'un antigène, lequel adjuvant comprend :
un tétra-polyol non toxique en une quantité de 0,2 à 49%;
une huile métabolisable non toxique en une quantité de 0 à 15%;
un tensio-actif à base d'un éther de glycol en une quantité de 0,05 à 5%;
de l'eau ou une solution aqueuse; et
0,0001 à 10% d'un dérive de muramyldipeptide de formule I.

9. Adjuvant selon la revendication 8, sous forme d'une émulsion de type huile-dans-eau, contenant des particules huileuses en dispersion dans une phase aqueuse continue, destiné à potentialiser l'immunogénicité d'un antigène, lequel adjuvant comprend :
du Tetronic® 1501 en une quantité de 1 à 10%;
du squalane ou du squalène en une quantité de 1 à 10%;
du Tween® 80 en une quantité de 0,2% environ;
du tampon salin isotonique; et
0,0001% à 10% de N-acétylmuramyl-L-thréonyle-D-iso-glutamine.

10. Vaccin sous forme d'une émulsion de type huile dans l'eau, contenant des particules en dispersion dans une phase aqueuse continue, destiné à imuuniser un animal, lequel vaccin comprend :
une quantité immunogène d'un antigène; et
un adjuvant selon l'une quelconque des revendication 1 à 9.

11. Procédé de préparation de l'adjuvant selon l'une quelconque des revendications 1 à 9, lequel procédé comprend le mélange de la phase aqueuse et de la quantité émulsifiante du tétra-polyol non toxique de manière à obtenir une émulsion.

12. Procédé selon la revendication 11, dans lequel le glycopeptide est ajouté au mélange suite à la formation de l'émulsion.

13. Procédé de préparation du vaccin selon la revendication 10, lequel procédé comprend le mélange de l'antigène avec l'adjuvant.

14. Trousse pour la préparation expresse d'un adjuvant selon l'une quelconque des revendications 1 à 9, laquelle trousse comprend :
un premier récipient contenant l'émulsion de tétra-polyol dans la phase aqueuse; et
un second récipient contenant le glycopeptide.

15. Trousse selon la revendication 14, pour la préparation expresse d'un adjuvant selon l'invention, laquelle trousse comprend :
un premier récipient contenant une émulsion de type huile-dans-eau, contenant des particules huileuses en dispersion dans une phase aqueuse continue, où ladite émulsion comprend du Tetronic® 1501, du squalane ou du squalène, du Tween® 80, et un tampon salin isotonique; et
un second récipient contenant de la N-acétylmuramyl-L-thréonyle-D-isoglutamine;
où les concentrations des composants dans chaque récipient sont choisies de manière à ce que la combinaison des contenus de chacun des deux récipients donne une formulation comprenant du Tetronic® 1501 en une quantité de 1 à 30%, du squalane ou du squalène en une quantité de 1 à 30%, du Tween® 80 en une quantité de 0,2 à 5% environ, 0,0001% à 30% de N-acétylmuramyl-L-thréonyl-D-isoglutamine, et un tampon salin isotonique.

16. Trousse pour la préparation expresse d'un vaccin selon la revendication 10, laquelle trousse comprend :
un premier récipient contenant l'émulsion de tétra-polyol en phase aqueuse; et
un second récipient contenant l'antigène:
dans laquelle le glycopeptide peut être incorporé dans un troisième récipient; ou dans le premier ou le second récipient.

17. Trousse selon la revendication 16, pour la préparation expresse d'un vaccin selon l'invention, laquelle trousse comprend :
un premier récipient contenant une émulsion de type huile-dans-eau, renfermant des particules huileuses en dispersion dans une phase aqueuse continue, où ladite émulsion comprend du Tetronic® 1501, du squalane ou du squalène, du Tween® 80, et un tampon salin isotonique; et
un second récipient contenant de la N-acétylmuramyl-L-thréonyl-D-isoglutamine et une quantité immunogène d'antigène;
où les concentrations des composants dans chacun des récipients sont choisies de manière à ce que la combinaison des contenus de chacun des deux récipients donne une formulation comprenant du Tetronic® 1501 en une quantité de 1 à 10%, du squalane ou du squalène en une quantité de 1 à 10%, du Tween® 80 en une quantité de 0,2% environ, 0,0001% à 10% de N-acétylmuramyl-L-thréonyl-D-isoglutamine, une quantité immunogène d'antigène; et du tampon salin isotonique.
